# NEUE EUROPÄISCHE PATENTSCHRIFT

(11) **EP 0 941 227 B2**
(45) Veröffentlichungstag und Bekanntmachung des Hinweises auf die Entscheidung über den Einspruch: **14.10.2009**
(45) Hinweis auf die Patenterteilung: 19.05.2004
(21) Anmeldenummer: 97951233.2
(22) Anmeldetag: 18.11.1997
(51) Int. Cl.: C07D 417/06, C07D 493/04, C12P 17/08, A01N 43/78, A61K 31/425, C07D 313/00, C07D 303/00

(54) **EPOTHILON D, DESSEN HERSTELLUNG UND DESSEN VERWENDUNG ALS CYTOSTATISCHES MITTEL BZW. ALS PFLANZENSCHUTZMITTEL**
EPOTHILONE D, PRODUCTION PROCESS, AND ITS USE AS CYTOSTATIC AS WELL AS PHYTOSANITARY AGENT
EPOTHILONE D, MODE DE PREPARATION ET APPLICATION COMME AGENT CYTOSTATIQUE ET PHYTOSANITAIRE

(30) Priorität: 18.11.1996 DE 19647580; 25.02.1997 DE 19707506
(43) Veröffentlichungstag der Anmeldung: 15.09.1999
(60) Teilanmeldung: 03016552.6
(73) Patentinhaber: Gesellschaft für Biotechnologische Forschung mbH (GBF), 38124 Braunschweig (DE)
(72) Erfinder: REICHENBACH, Hans, D-38124 Braunschweig (DE); HÖFLE, Gerhard, D-38124 Braunschweig (DE); GERTH, Klaus, D-38124 Braunschweig (DE); STEINMETZ, Heinrich, D-38124 Braunschweig (DE)
(74) Vertreter: Boeters, Hans Dietrich
(86) Internationale Anmeldenummer: PCT/EP1997/006442
(87) Internationale Veröffentlichungsnummer: WO 1998/022461

(56) Entgegenhaltungen:
- WO-A-93/10121
- WO-A-97/19086
- WO-A-98/08849
- BALOG A. ET AL.: "Total synthesis of (-)-epothilone A" ANGEWANDTE CHEMIE, INTERNATIONAL EDITION IN ENGLISH, Bd. 35, Nr. 23/24, 3.Januar 1997, Seiten 2801-2803, XP002035359
- NICOLAOU K.C. ET AL.: "An approach to epothilones based on olefin metathesis" ANGEWANDTE CHEMIE, INTERNATIONAL EDITION IN ENGLISH, Bd. 35, Nr. 20, 4.November 1996, Seiten 2399-2401, XP002035372

## Beschreibung

Die vorliegende Erfindung betrifft Epothilone D, dessen Herstellung sowie ein therapeutisches Mittel.

Gemäß einer Ausführungsform betrifft die Erfindung ein Verfahren zur Herstellung von Epothilon [ D], bei dem man
(a) *Sorangium cellulosum* DSM 6773 in Gegenwart eines Adsorberharzes in an sich bekannter Weise kultiviert;
(b) das Adsorberharz von der Kultur abtrennt und mit einem Wasser/Methanol-Gemisch wäscht,
(c) das gewaschene Adsorberharz mit Methanol eluiert und das Eluat zu einem Rohextrakt einengt,
(d) das gewonnene Konzentrat mit Ethylacetat extrahiert, den Extrakt einengt und zwischen Methanol und Hexan verteilt,
(e) die methanolische Phase zu einem Raffinat einengt und das Konzentrat an einer Sephadex-Säule fraktioniert,
(f) eine Fraktion mit Stoffwechselprodukten des eingesetzten Mikroorganismus gewinnt,
(g) die gewonnene Fraktion an einer C18-Umkehrphase mit einem Methanol/Wasser-Gemisch chromatographiert und in zeitlicher Reihenfolge
   - nach einer ersten Fraktion mit Epothilon A und
   - einer zweiten Fraktion mit Epothion B
   - eine dritte Fraktion mit einem ersten weiteren Epothilon (Epothilon C) und
   - eine vierte Fraktion mit einem zweiten weiteren Epothilon (Epothilon D) gewinnt und
(h) das Epothilon der zweiten weiteren Fraktion (Epothilon D) isoliert.

Ferner betrifft die Erfindung Epothilon D der Formel: 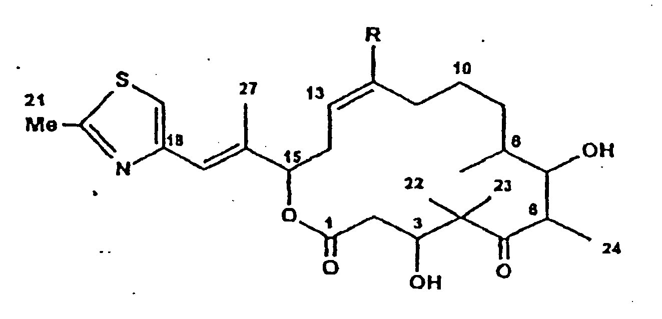 Epothilon D R = CH₃
und der Summenformel C₂₇H₄₁NO₅S, gekennzeichnet durch das ¹H- und ¹³C-NMR-Spektrum gemäß Tabelle 1. Epothilon D kann zur Herstellung der Verbindungen der folgenden **Formel 1** verwendet werden, wobei zu deren Derivatisierung auf die in WO-A-97/19 086 beschriebenen Derivatisierungsmethoden verwiesen werden kann.

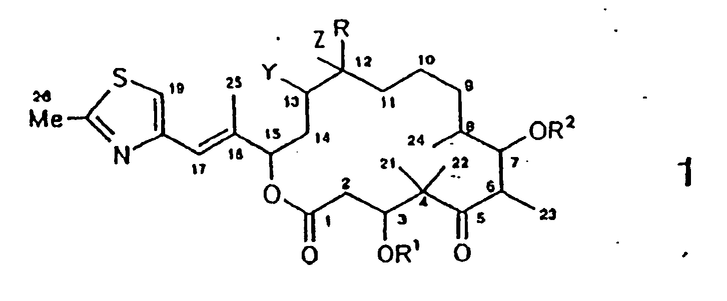

In der vorstehenden Formel 1 bedeuten:
R = H, C₁₋₄-Alkyl;
R¹, R², R³, R⁴, R⁵ = H, C₁₋₆-Alkyl,
C₁₋₆-Acyl-Benzoyl,
C₁₋₄-Trialkylsilyl,
Benzyl,
Phenyl,
C₁₋₆-Alkoxy-,
C₆-Alkyl-, Hydroxy- und Halogensubstituiertes Benzyl bzw. Phenyl;
wobei auch zwei der Reste R¹ bis R⁵ zu der Gruppierung -(CH₂)ₙ- mit n = 1 bis 6 zusammentreten können und es sich bei den in den Resten enthaltenen Alkyl- bzw. Acylgruppen um gradkettige oder verzweigte Reste handelt;
Y und Z sind entweder gleich oder verschieden und stehen jeweils für Wasserstoff, Halogen, wie F, Cl, Br oder J, Pseudohalogen, wie -NCO, -NCS oder -N₃, OH, O-(C₁₋₆)-Acyl, O-(C₁₋₆)-Alkyl, O-Benzoyl. Y und Z können auch das O-Atom eines Epoxides sein, oder eine der C-C-Bindungen einer C=C-Doppelbindung bilden.

So kann man die 12,13-Doppelbindung selektiv
- hydrieren, beispielsweise katalytisch oder mit Diimin, wobei man eine Verbindung der Formel 1 mit Y = Z = H erhält; oder
- epoxidieren, beispielsweise mit Dimethyldioxiran oder einer Persäure, wobei man eine Verbindung der **Formel 1** mit Y mit Z = -O- erhält; oder
- in die Dihalogenide, Dipseudohalogenide oder Diazide umwandeln, wobei man eine Verbindung der **Formel 1** mit Y und Z = Hal, Pseudo-hal oder N₃ erhält.

### Herstellung und Mittel

Schließlich betrifft die Erfindung ein therapeutisches Mittel, insbesondere zum Einsatz als Cytostatikum, bestehend aus Epothilon D oder aus Epothilon D neben einem oder mehreren üblichen Träger(n) und/oder Verdünnungsmittel(n).

Die Erfindung wird im folgenden durch die Beschreibung von einigen ausgewählten Ausführungsbeispielen näher erläutert und beschrieben.

### Beispiele

### Beispiel 1:

### Epothilon D

### A. Produktionsstamm und Kulturbedingungen entsprechend dem Epothilon Basispatent DE-B-41 38 042.

### B. Produktion mit DSM 6773

75 l Kultur werden wie im Basispatent beschrieben angezogen und zum Animpfen eines Produktionsfermenters mit 700 l Produktionsmedium aus 0.8 % Stärke, 0.2 % Glukose, 0.2 % Soyamehl, 0.2 % Hefeextrakt, 0.1 % CaCl₂ x 2H₂O, 0.1 % MgSO₄ x 7H₂O, 8 mg/l Fe-EDTA, pH = 7.4 und optional 15 l Adsorberharz Amberlite® XAD-16 verwendet. Die Fermentation dauert 7 - 10 Tage bei 30 C, Belüftung mit 0.1 NL/m³. Durch Regulierung der Drehzahl wird der pO₂ bei 30 % gehalten.

### C. Isolierung

Das Adsorberharz wird mit einem 0.7 m², 100 mesh Prozeßfilter von der Kultur abgetrennt und durch Waschen mit 3 Bettvolumen Wasser/Methanol 2:1 von polaren Begleitstoffen befreit. Durch Elution mit 4 Bettvolumen Methanol wird ein Rohextrakt gewonnen, der i. Vak. bis zum Auftreten der Wasserphase eingedampft wird.

Diese wird dreimal mit dem gleichen Volumen Ethylacetat extrahiert. Eindampfen der organischen Phase ergibt 240 g Rohextrakt, der zwischen Methanol und Heptan verteilt wird, um lipophile Begleitstoffe abzutrennen. Aus der Methanolphase werden durch Eindampfen i. Vak 180 g Raffinat gewonnen, das in drei Portionen über Sephadex® LH-20 (Säule 20 x 100 cm, 20 ml/min Methanol) fraktioniert wird. Die Epothilone sind in der mit 240 - 300 min Retentionszeit eluierten Fraktion von insgesamt 72 g enthalten. Zur Trennung der Epothilone wird in drei Portionen an Lichrosorb® RP-18 (15 µm, Säule 10 x 40 cm, Laufmittel 180 ml/min Methanol/Wasser 65:35) chromatographiert. Nach Epothilon A und B werden mit Rₜ = 90-95 min Epothilon C und 100-110 min Epothilon D eluiert und nach Eindampfen i. Vak. in einer Ausbeute von jeweils 0.3 g als farblose Öle gewonnen.

### D. Physikalische Eigenschaften

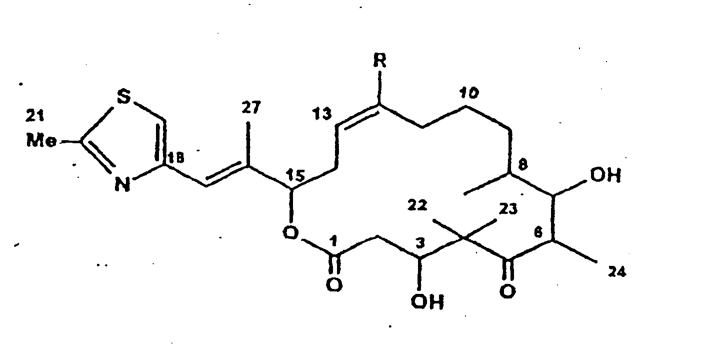

### Epothilon D R = CH₃

### Epothilon D

C₂₇H₄₁NO₅S [491]
ESI-MS: (positiv Ionen) : 492,5 für [M+H]⁺
1H und 13C siehe NMR-Tabelle
DC: R_{f} = 0,82
DC-Alufolie 60 F 254 Merck, Laufmittel: Dichlormethan/Methanol = 9:1
Detektion: UV-Löschung bei 254 nm. Ansprühen mit Vanillin-Schwefelsäure-Reagenz, blau-graue Anfärbung beim Erhitzen auf 120 °C.
HPLC: Rₜ = 15,3 min
Säule: Nucleosil® 100 C-18 7µm, 125 x 4 mm
Laufmittel: Methanol/Wasser = 65:35
Fluß: 1ml/min
Detection: Diodenarray

**Tabelle 1:**

| **¹H- und ¹³C-NMR Daten von Epothilon D in [D₆] DMSO bei 300 MHz** | | | |
|---|---|---|---|
| H-Atom | Epothilon D | | |
| | δ (ppm) | C-Atom | δ (ppm) |
| | | 1 | 170.1 |
| 2-Ha | 2.35 | 2 | 39.0 |
| 2-Hb | 2.38 | 3 | 70.8 |
| 3-H | 4.10 | 4 | 53.2 |
| 3-OH | 5.08 | 5 | 217.4 |
| 6-H | 3.11 | 6 | 44.4 |
| 7-H | 3.48 | 7 | 75.5 |
| 7-OH | 4.46 | 8 | 36.3 |
| 8-H | 1.29 | 9 | 29.9 |
| 9-Ha | 1.14 | 10 | 25.9 |
| 9-Hb | 1.38 | 11 | 31.8* |
| 10-Ha | 1.14* | 12 | 138.3 |
| 10-Hb | 1.35* | 13 | 120.3 |
| 11-Ha | 1.75 | 14 | 31.6* |
| 11-Hb | 2.10 | 15 | 76.6 |
| 12-H | | 16 | 137.2 |
| 13-H | 5.08 | 17 | 119.2 |
| 14-Ha | 2.30 | 18 | 152.1 |
| 14-Hb | 2.65 | 19 | 117.7 |
| 15-H | 5.29 | 20 | 164.3 |
| 17-H | 6.51 | 21 | 18.9 |
| 19-H | 7.35 | 22 | 19.7 |
| 21-H₃ | 2.65 | 23 | 22.5 |
| 22-H₃ | 0.90 | 24 | 16.4 |
| 23-H₃ | 1.19 | 25 | 18.4 |
| 24-H₃ | 1.07 | 26 | 22.9 |
| 25-H₃ | 0.91 | 27 | 14.1 |
| 26-H₃ | 1.63 | | |
| 27-H₃ | 2.11 | | |

| | | | |
|---|---|---|---|
| * Zuordnung vertauschbar | | | |

### Beispiel 2 und Vergleichsbeispiele 1 bis 5:

Das erfindungsgemäße Epothilon D und Epothilone A, B, C, E und F (Vergleichsbeispiele 1 bis 5) wurden mit Zellkulturen (Tabelle 2) und auf Polymerisationsförderung (Tabelle 3) getestet.

**Tabelle 2:**

| **Epothilon-Tests mit Zellkulturen** | | | | | | |
|---|---|---|---|---|---|---|
| Epothilon | A 493 | B 507 IC-50 | C 477 [ng/ml] | D 491 | E 509 | F 523 |
| Mausfibroblasten L 929 | 4 | 1 | 100 | 20 | 20 | 1,5 |
| humane Tumorzellinien: | | | | | | |
| HL-60 (Leukämie) | 0.2 | 0.2 | 10 | 3 | 1 | 0,3 |
| K-562 (Leukämie) | 0.3 | 0.3 | 20 | 10 | 2 | 0,5 |
| U-937 (Lymphom) | 0.2 | 0.2 | 10 | 3 | 1 | 0,2 |
| KB-3.1 (Cervixkarzinom) | 1 | 0.6 | 20 | 12 | 5 | 0,5 |
| KB-V1 (Cervixkarzinom multires | 0.3 | 0.3 | 15 | 3 | 5 | 0,6 |
| A-498 (Nierenkarzinom) | - | 1.5 | 150 | 20 | 20 | 3 |
| A-549 (Lungenkarzinom) | 0.7 | 0.1 | 30 | 10 | 3 | 0,1 |

**Tabelle 3:**

| **Polymerisationstest mit Epothilonen** | | | | | | |
|---|---|---|---|---|---|---|
| Parameter: Zeit bis zur halbmaximalen Polymerisation der Kontrolle | | | | | | |

| Messung: | w | x | y | z | **Mittel** | **Mittel** |
|---|---|---|---|---|---|---|
| | | | | | **[s]** | **[%]** |
| Kontrolle | 200 | 170 | 180 | 210 | **190** | **100** |
| Epothilon A | 95 | 60 | 70 | 70 | **74** | **39** |
| Epothilon B | | 23 | 25 | 30 | **26** | **14** |
| Epothilon C | 125 | 76 | 95 | 80 | **94** | **49** |
| Epothilon D | 125 | 73 | 120 | | **106** | **56** |
| Epothilon E | 80 | 60 | 50 | 45 | **59** | **31** |
| Epothilon F | 80 | 40 | 30 | 50 | **50** | **26** |

### Standardtest mit 0,9 mg Tubulin/ml und 1 µM Probenkonzentration

Der Polymerisationstest ist ein in vitro Test mit gereinigtem Tubulin aus Schweinehirn. Die Auswertung erfolgt photometrisch. Polymerisationsfördernde Substanzen wie die Epothilone verkürzen die Zeit, bis zu der halbmaximale Polymerisation erfolgt ist, d. h., je kürzer die Zeit, desto wirksamer die Verbindung. w, x, y und z sind vier unabhängige Versuche, die relative Wirksamkeit ist in der letzten Spalte in % der Kontrolle ausgedrückt; wieder zeigen die niedrigsten Werte die beste Wirksamkeit an. Die Rangliste entspricht ziemlich genau der in Zellkulturen festgestellten.

## Patentansprüche

1. Verfahren zur Herstellung von Epothilon D, bei dem man
(a) *Sorangium cellulosum* DSM 6773 in Gegenwart eines Adsorberharzes in an sich bekannter Weise kultiviert,
(b) das Adsorberharz von der Kultur abtrennt und mit einem Wasser/Methanol-Gemisch wäscht,
(c) das gewaschene Adsorberharz mit Methanol eluiert und das Eluat zu einem Rohextrakt einengt,
(d) das gewonnene Konzentrat mit Ethylacetat exrahiert, den Extrakt einengt und zwischen Methanol und Hexan verteilt,
(e) die methanolische Phase zu einem Raffinat einengt und das Konzentrat an einer Sephadex-Säule fraktioniert,
(f) eine Fraktion mit Stoffwechselprodukten des eingesetzten Mikroorganismus gewinnt,
(g) die gewonnene Fraktion an einer C18-Umkehrphase mit einem Methanol/Wasser-Gemisch chromatographiert und in zeitlicher Reihenfolge
- nach einer ersten Fraktion mit Epothilon A und
- einer zweiten Fraktion mit Epothilon B
- eine dritte Fraktion mit einem ersten weiteren Epothilon (Epothilon C) und
- eine vierte Fraktion mit einem zweiten weiteren Epothilon (Epothilon D) gewinnt und
(h) das Epothilon (Epothilon D) der zweiten weiteren Fraktion isoliert.

2. Epothilon D der Formel: 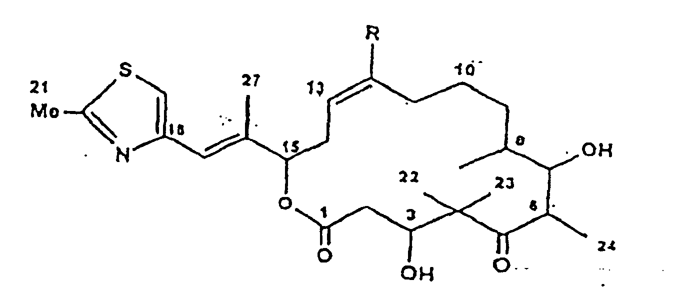 Epothilon D R = CH₃ und der Summenformel C₂₇H₄₁NO₅S, **gekennzeichnet durch** das ¹H- und ¹³C-NMR-Spektrum gemäß Tabelle 1.

3. Therapeutisches Mittel, insbesondere zum Einsatz als Cytostatikum, bestehend aus einer Verbindung nach Ansprüche 2 oder einer Verbindung nach Ansprüche 2 neben einem oder mehreren üblichen Träger(n) und/oder Verdünnungsmittel(n).

## Claims

1. Process for the preparation of epothilone D, wherein
(a) Sorangium cellulosum DSM 6773 is cultured in a manner known per se in the presence of an adsorber resin,
(b) the adsorber resin is removed from the culture and washed with a water/methanol mixture,
(c) the washed adsorber resin is eluted with methanol and the eluate is concentrated to give a crude extract,
(d) the concentrate obtained is extracted with ethyl acetate, the extract is concentrated and partitioned between methanol and hexane,
(e) the methanolic phase is concentrated to give a raffinate and the concentrate is fractionated on a Sephadex column,
(f) a fraction containing metabolic products of the microorganism employed is obtained,
(g) the fraction obtained is chromatographed on a C18 reverse phase with a methanol/water mixture and, sequentially
- after a first fraction containing epothilone A and
- a second fraction containing epothilone B
- a third fraction containing a first further epothilone (epothilone C) and
- a fourth fraction containing a second further epothilone (epothilone D) are obtained and
(h) the epothilone (epothilone D) of the second further fraction is isolated.

2. Epothilone D of the formula: 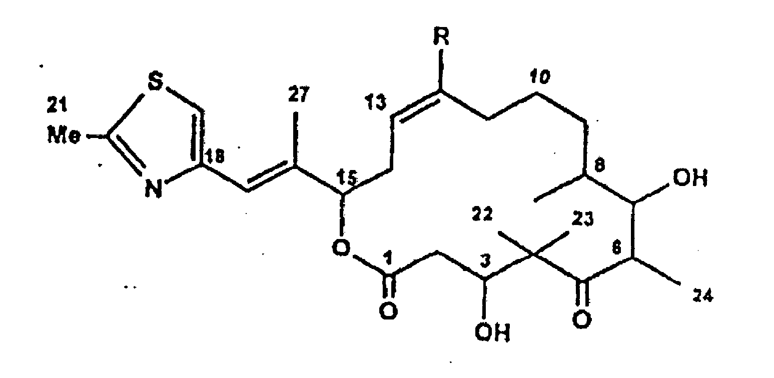 Epothilone D R = CH₃
and the empirical formula C₂₇H₄₁NO₅S, **characterized by** the ¹H- and ¹³C-NMR spectrum as in Table 1.

3. Therapeutic composition, in particular for use as a cytostatic, consisting of a compound according to claim 2 or a compound according to claim 2 in combination with one or more customary carrier(s) and/or diluents(s)

## Revendications

1. Procédé de préparation de l'épothilone D, dans lequel :
(a) on cultive d'une manière connue en soi *Sorangium cellulosum* DSM 6773 en présence d'une résine adsorbante,
(b)on sépare la résine adsorbante de la culture et on la lave avec un mélange d'eau et de méthanol,
(c) on élue la résine adsorbante lavée avec du méthanol et on concentre l'éluat en un extrait brut,
(d)on extrait le concentré obtenu avec de l'acétate d'éthyle, on concentre l'extrait et on le partage entre du méthanol et de l'hexane,
(e)on concentre la phase méthanolique en un produit de raffinage et on fractionne le concentré sur une colonne de Séphadex,
(f) on obtient une fraction avec des produits du métabolisme du microorganisme utilisé,
(g)on chromatographie la fraction obtenue sur une phase d'inversion C18 avec un mélange de méthanol et d'eau et on obtient dans l'ordre chronologique :
- après une première fraction contenant de l'épothilone A et une deuxième fraction contenant de l'épothilone B,
- une troisième fraction contenant une première autre épothilone (épothilone C) et une quatrième fraction contenant une deuxième autre épothilone (épothilone D), et
(h)on isole l'épothilone (épothilone D) de la deuxième autre fraction.

2. Epothilone D de formule : 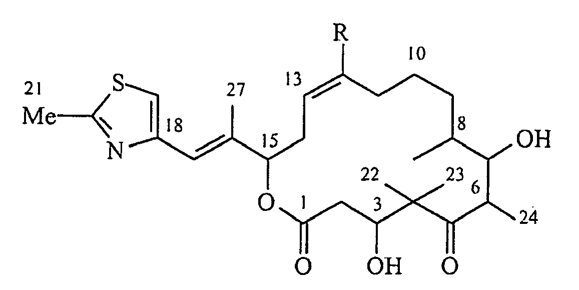 Epothilone D R=CH₃
et de formule brute C₂₇H₄₁NO₅S **caractérisée par** les spectres de RMN de ¹H et ¹³C selon le Tableau 1.

3. Agent thérapeutique, utilisable en particulier comme agent cytostatique, qui est constitué d'un composé selon la revendication 2, ou d'un composé selon la revendication 2 et d'un ou plusieurs supports et/ou diluants habituels.
